Europäisches Patentamt

European Patent Office

Office européen des brevets

(19)

(11) **EP 1 183 056 B1**

(12) **EUROPÄISCHE PATENTSCHRIFT**

(45) Veröffentlichungstag und Bekanntmachung des Hinweises auf die Patenterteilung:
**21.01.2004 Patentblatt 2004/04**

(21) Anmeldenummer: **00925295.8**

(22) Anmeldetag: **19.05.2000**

(51) Int Cl.$^7$: **A61L 15/26**, A61L 15/52

(86) Internationale Anmeldenummer:
**PCT/EP2000/004531**

(87) Internationale Veröffentlichungsnummer:
**WO 2000/074739 (14.12.2000 Gazette 2000/50)**

(54) **POLYURETHANTRÄGERFOLIE MIT HYDROPHOBIERUNGSMITTELN FÜR FILMPFLASTER**

POLYURETHANE SUPPORT FILM PROVIDED WITH HYDROPHOBIC AGENTS FOR FILM PLASTERS

FEUILLE SUPPORT EN POLYURETHANNE POURVUE D'AGENTS IMPERMEABILISANTS POUR PANSEMENT PELLICULAIRE

(84) Benannte Vertragsstaaten:
**AT BE CH CY DE DK ES FI FR GB GR IE IT LI LU MC NL PT SE**

(30) Priorität: **08.06.1999 DE 19925972**

(43) Veröffentlichungstag der Anmeldung:
**06.03.2002 Patentblatt 2002/10**

(73) Patentinhaber: **Beiersdorf AG**
**20253 Hamburg (DE)**

(72) Erfinder:
• **BRUSS, Witta**
  **D-86199 Augsburg (DE)**
• **GÖTZ, Gabriela**
  **D-22395 Hamburg (DE)**
• **MAYAN, Robert**
  **D-21614 Buxtehude (DE)**

(56) Entgegenhaltungen:
| | |
|---|---|
| **EP-A- 0 409 587** | **EP-A- 0 815 880** |
| **DE-A- 4 314 834** | **GB-A- 2 207 867** |
| **US-A- 5 643 187** | **US-A- 5 679 754** |
| **US-A- 5 757 735** | |

**Beschreibung**

Die Erfindung betrifft Filmpflaster insbesondere zur Abdeckung von Wunden und Verhütung oder Behandlung von Blasen unter Verwendung von Trägerfolien mit verbesserten Oberflächeneigenschaften.

Filme und Folien finden in Pflastern und Wundschnellverbänden aufgrund ihrer Wasserundurchlässigkeit, ihrer Keimdichtigkeit, ihrer Anschmiegsamkeit und ihrer hohen Verträglichkeit häufig Verwendung.

So offenbart die DE 43 14 834 A1 ein Verbandmaterial auf Folienbasis, das auf der einen Seite mit einem Trägermaterial abgedeckt ist, das die gleiche Größe wie die Folie besitzt und mindestens eine Griffleiste aufweist, und das auf der anderen Seite mit einer selbstklebenden Schicht versehen ist. Erfindungswesentlich ist hier, daß die Griffleisten innerhalb der Umfassungsbegrenzung des Trägermaterials angeordnet sind. Vorzugsweise ist nur eine Griffleiste auf dem Trägermaterial angebracht.
Ein solches Pflaster mit Polyurethanfolie ist im Handel unter dem Namen "Aqua Protect" ® von der Firma Beiersdorf erhältlich.

GB 2207 867 beschreibt eine Wundauflage, die aus einer Polyurethan-Film zusammen mail mit einer hydrophoben Komponente besteht. Als bevorzugte hydrophobe Komponenten sind Siloxon Polymere genannt.

Die DE 40 26 755 A1 offenbart ein Verbandmaterial auf Folienbasis, das auf der einen Seite mit einem Stützmaterial abgedeckt ist, das die gleiche Größe wie die Folie besitzt und mindestens eine Griffleiste aufweist, und auf der anderen Seite mit einer selbstklebenden Schicht versehen ist. Die Griffleisten zur Entfernung des Trägermaterials sind im Gegensatz zu dem Verbandmaterial gemäß DE 43 14 834 C2 innerhalb der Umfassungsbegrenzung des Trägermaterials angeordnet. Vorzugsweise ist auch hier nur eine Griffleiste auf dem Trägermaterial angebracht.
Im Handel kann dieses Pflaster mit Polyurethanfolie unter dem Namen "Cutifilm" ® ebenfalls von der Firma Beiersdorf bezogen werden.

Bei medizinischen Pflastern, Wundverbänden und Fixierungen aller Art tritt häufig ein Phänomen auf, das zu einer vorzeitigen, unbeabsichtigten Ablösung führt. Dieses Phänomen ist ein Aufrollen des Produkts, meist von einer Ecke oder aber einer Kante des Pflasters ausgehend.

Hat sich das Pflaster an einem Punkt gelöst, folgt eine Kettenreaktion, die sehr rasch zu einem vollständigen Ablösen führt. Besonders oft findet dieses Aufrollen bei Pflastern statt, die unter Kleidung oder in Schuhen getragen werden. Ursache ist die Reibung des Kleidungsstücks oder Schuhs an der Oberfläche des Pflasters. Diese Reibungskraft verursacht eine dynamische Scherbelastung der Haftklebemasse, welche meist sehr schnell zu einem Bruch der Verklebung im Randbereich führt. Nach dem Lösen der Klebmasse an einer Kante haftet das Textil oder Leder an der abstehenden Masse und verursacht durch die tangential anliegende Kraft ein Aufrollen und weiteres, beschleunigtes Ablösen des gesamten Pflasters.

Eine Möglichkeit zur Vermeidung des vorzeitigen Ablösens ist die Erhöhung der Adhäsion der Haftklebemasse auf der Haut. Diese Klebrigkeit läßt sich aber nicht beliebig erhöhen, da es sonst beim beabsichtigten Ablösen des Produkts zu Hautreizungen, Schmerzempfinden und Störung der Wundruhe kommen kann.

Aus der EP 0 409 587 A1 ist bekannt, wie stark das vorzeitige Ablösen von Pflastern von der Kontaktfläche A, also jener Fläche, auf der sich zwei gleitende Körper tatsächlich berühren, der Trägerfolie beeinflußt wird.
Dort wird die Verwendung thermoplastischer Folien beschrieben, die beim oder nach dem Extrudieren in geschmolzenem Zustand durch eine Prägewalze hohlgeprägt werden. Beste Ergebnisse werden mit einer Struktur erhalten, bei der die Kontaktfläche ca. 25% der Gesamtfläche darstellt.

Die Verwendung von Folien zur Bildung von Pflastern, hergestellt unter Verwendung von Hydrophobierungsmitteln ist nicht Gegenstand der Offenbarung.

Nach US 5,643,187 werden Pflaster und Wundschnellverbände mit guter Gleitfähigkeit und gleichzeitiger guter Elastizität durch einen zweischichtigen Aufbau der Trägerfolie erhalten. Niedrige Gleitreibung wird dabei erzielt durch Aufbringen einer dünnen Schicht eines vergleichsweise harten Kunststoffs, während die Anschmiegsamkeit und Elastizität durch Verwendung einer dickeren Schicht aus vergleichsweise weichem, dehnbarem Material sichergestellt werden soll. Nach der Applikation des Pflasters stellt die harte, gleitfähige Seite des Trägerfilms die äußere, der Haut abgewandte Oberfläche dar.
Nachteil dieser Methode zur Verbesserung der Gleitfähigkeit ist eine unvermeidliche Einbuße von Dehnbarkeit, Elastizität und damit Anschmiegsamkeit der Trägerfolie. Zur Dehnung von harten Folien oder Folienschichten muß deutlich mehr Kraft aufgebracht werden, so daß es bei der Anwendung als Pflaster zu Hautirritationen und verzögertem Heilungsverlauf aufgrund mechanischer Belastung der Wunde kommen kann.

Eine Methode zur Beschichtung von medizinischen Artikeln, insbesondere von Untersuchungs- und OP-Handschuhen, beschreibt US 5,742,943. Verwendung findet hier ein komplexes Gemisch aus verschiedenen Chemikalien wie kationischen Tensiden, insbesondere 1-Hexadecylpyridin-hydrochlorid, Acetylendiol-Verbindungen und modifizierten Siliconen. Ziel dieser Beschichtung ist eine verbesserte Gleitfähigkeit auf trockener oder feuchter Haut. Bei den hierzu eingesetzten Chemikalien handelt es sich nicht um Hydrophobierungsmittel.

Die Verwendung von elastischen, gleitfähigen Folien zur Bildung von Pflastern und Wundschnellverbänden, hergestellt durch Ausrüstung eines hydrophilen Polyurethanfilms mit Hydrophobierungsmitteln ist nicht Gegenstand der oben genannten Patentschriften.

Die Reibungskraft $F_r$ ist nach dem ersten Gesetz der Reibung gleich dem Produkt aus Reibungskoeffizient $\mu$ und Normalkraft $F_n$. Dieser Koeffizent ist ein Maß für die Kraft, die man aufwenden muß, um einen Körper auf einer Oberfläche zu bewegen, wobei $\mu_s$ den statischen (Haftreibung) und $\mu_k$ den kinetischen (Gleitreibung) Reibungskoeffizienten bezeichnet.

Die Entwicklung von Trägem mit gutem Gleitvermögen, d.h. niedrigen Reibungskoeffizienten, ist demnach ein zentraler Ansatzpunkt, um den oben geschilderten Effekt des Aufrollens zu vermeiden. Obwohl, wie vor allem von Ludema (Ludema, K.C., Friction, Wear, Lubrication: a Textbook in Tribology, CRC Press, Boca Raten 1996) dargestellt, bisher weder exakte noch näherungsweise Methoden existieren, um Reibungs- oder Abnutzungsverhalten aus grundlegenden Prinzipien abzuleiten, erlaubt eine Durchsicht der Literatur Rückschlüsse auf Parameter, welche die Größe von $\mu_s$ und $\mu_k$ bestimmen.

Für die Haftreibung gilt (Blau, PJ.; Friction Science and Technology, Marcel Dekker, New York 1996) folgender Ausdruck:

$$\mu_s = (\tau_m/P^*)\ A$$

wobei

$\tau_m$ die Scherfestigkeit,
A die Kontaktfläche und
P* die Kombination von Normalkraft und Adhäsion

bezeichnet

Die Gleitreibung zwischen zwei Körpern wird von einer Reihe von Effekten bestimmt, die zusammenwirken (Bhushan, B., Gupta, B.K.; Handbook of Tribology, McGraw-Hill New York 1991). Neben Adhäsionskomponenten treten Plowing-, Rauhigkeits-, Deformationsund, besonders bei viskoelastischen Materialien, Dämpfungseffekte auf. Der relative Beitrag dieser Effekte hängt von den beteiligten Materialien, der Oberflächentopographie, dem Zustand der gleitenden Oberflächen und den Umgebungsbedingungen ab.

Aus Untersuchungen von Bartenev (Bartenev, G.M., Lavrentev, V.V.; Friction and Wear of Polymers, Elsevier Amsterdam 1981) und Rabinowicz (Rabinowicz, E.; Friction and Wear of Materials, Wiley-Interscience, New York 1995) geht hervor, daß neben der Kontaktfläche Parameter wie Rauhigkeit, Härte, Elastizitätsmodul und Oberflächenenergie der Materialien den Reibungskoeffizienten $\mu_k$ bestimmen.

Der Einfluß von Fluor Polymeren auf den Reibungskoeffizienten von Kunststoffen gegen Stahl wurde von Mens und de Gee (Mens, J.W.E., de Gee, A.W.J.; Friction and wear behaviour of 18 polymers in contact with steel in environments of air and water, Wear **149**, 255 bis 268 (1991)) an Polytetrafluorethen als Additiv untersucht.

Nachfolgende Tabelle zeigt die Werte für Kunststoff gegen AISI 52100 Stahl mit 0,1 m/s und 500 N Belastung ohne beziehungsweise mit Zusatz von PTFE.

Tabelle 1:

| Auswirkung von PTFE Additiv auf die Reibungskoeffizienten von Polymer gegen Stahl | | |
|---|---|---|
| Basispolymer | $\mu$ (Basispolymer) | $\mu$ (mit 15 % PTFE) |
| Polyamid 66 | 0,57 | 0,13 |
| Polyoxymethylene (POM) | 0,45 | 0,21 |
| Polyether-ether-keton (PEEK) | 0,49 | 0,18 |
| Polyethylenterephtalat (PET) | 0,68 | 0,14 |
| Polyphenylenesulphid (PPS) | 0,70 | 0,30 |
| Polyetherimid (PEI) | 0,43 | 0,21 |

Aufgabe der Erfindung ist es, die aus dem Stand der Technik bekannten Nachteile zu vermeiden und ein Filmpflaster zur Verfügung zu stellen, das gegebenenfalls einseitig selbstklebend ausgerüstet ist und das sich nicht unbeabsichtigt von der Hautstelle löst, auf der es zuvor verklebt worden ist. Weiterhin wird durch erfindungsgemäße Ausführung des Filmpflasters die Anschmutzbarkeit verringert und die Wasserabweisung verbessert. Eine verbesserte Wasserabweisung von PU-Filmen ist vor allem von großem Interesse, wenn Polyurethan-Typen mit sehr hohen Wasserdampfdurchlässigkeiten eingesetzt werden. Diese Typen weisen besonders hohe Hydrophilie auf, so daß die wasserabweisenden Eigenschaften der Filmoberfläche durch Hydrophobierungsmittel stark erhöht werden kann, während die Wasserdampfdurchlässigkeit des gesamten Films nicht beeinträchtigt wird.

Gelöst wird diese Aufgabe durch ein Filmpflaster, wie es im Hauptanspruch niedergelegt ist. Gegenstand der Unteransprüche sind dabei vorteilhafte Weiterbildungen des Filmpflasters.

Demgemäß ist Gegenstand der vorliegenden Erfindung die Verwendung von zumindest einer hydrophilen Polyurethan-Folie insbesondere als Träger für medizinische Pflaster und Wundschnellverbände, wobei der Träger insbesondere durch Ausstreichen verdickter Polyurethan-Dispersionen auf Casting-Papieren oder -Folien hergestellt wird.

Die Anmutung und Oberflächenstruktur der Folien wird durch Wahl des Casting-Substrats festgelegt.

Das Filmpflaster bestehend aus zumindest einem elastischen Polyurethan-Film, der auf der der Hant abgewandten Oberflächenseite mit Hydrophobierungsmitteln auf Fluorcarbon-, Silicon- oder Kohlenwasserstoff-Basis ausgerüstet ist und auf der Hant zugewandten seite mit einem Haftkleber beschichtet ist.

Durch Einsatz von Hydrophobierungsmittel bei der Herstellung von PU-Trägerfolien für Filmpflaster kann die Oberflächenenergie in einem sehr breiten Bereich gezielt variiert und minimiert werden. Die Oberflächenenergie des PU-Films ist für folgende Produkteigenschaften relevant:

- die Gleiteigenschaften des Films
- Anschmutzbarkeit des Filmpflasters
- Wasserabweisung des Films.

Bevorzugte Polyurethan-Dispersion zur Herstellung von medizinischen Pflastern zur Abdeckung von Wunden und Behandlung oder Verhütung von Blasen sind beispielsweise bei der Bayer AG, Leverkusen, unter dem Namen Impranil und Impraperm erhältlich. Diese Dispersionen können durch Zusatz geeigneter Additive aufgeschäumt werden, so daß sich auch Schäume als Träger herstellen lassen. Durch Mischen verschiedener Impranil- und/oder Impraperm-Qualitäten und optionale Herstellung mehrlagiger Schichten durch sukzessives Ausstreichen verschiedener geschäumter oder ungeschäumter Dispersionen auf einem Substrat können Trägermaterialien mit gewünschten Eigenschaften wie Härte, Elastizitätsmodul, Dehnbarkeit, Wasserdampfdurchlässigkeit, Rauhigkeit, Griff und Anmutung erzeugt werden. Die an sich farblosen Filme oder Schäume lassen sich durch Zusatz von handelsüblichen Pigmenten wie beispielsweise Euderm (Bayer AG, Leverkusen) einfärben.

In der einfachsten Ausführung besteht der Film aus einer Schicht.

Zur erfindungsgemäßen Optimierung der Oberflächenenergie des Films stehen zwei Verfahren zur Wahl:

Geeignete Additive wie beispielsweise die Fluor Verbindungen Xeroderm WF (Bayer AG, Leverkusen) werden in einer Menge bis 5 Gew.-% der Dispersion beigefügt. Bevorzugt ist das Hydrophobierungsmittel zu einem Anteil von 0,1 bis 5 Gew.-%, insbesondere 1 bis 5 Gew.-%, im Polyurethan-Film enthalten. Aufgrund der oberflächenaktiven Eigenschaften diffundiert diese Verbindung an die Grenzfläche und verringert dadurch im fertigen Produkt die Oberflächenenergie des Films.

Somit stellt sich ein bevorzugtes Verfahren zur Herstellung eines erfindungsgemäßen Filmpflasters wie folgt dar.

Zumindest eine Polyurethan-Dispersion, enthaltend ein Hydrophobierungsmittel zu einem Anteil von bis zu 5 Gew.-%, wird auf einem geprägten wasserfesten Silicon- oder Polypropylen-beschichteten Papier oder Folie ausgestrichen. Der Verbund wird getrocknet.

Der sich daraus ergebene Polyurethanfilm wird mit einer Haftklebemasse beschichtet, die Haftklebemasse gegebenenfalls mit einer Wundabdeckung und einem klebstoffabweisenden Trägermaterial versehen und das wasserfeste Silicon- oder Polypropylen-beschichtete Papier oder die Folie entfernt.

Aufgrund der hohen Hydrophilie des Polyurethan kann der getrocknete Film auch durch Eintauchen in beziehungsweise Besprühen oder Bestreichen mit wäßrigen Dispersionen von Hydrophobierungsmitteln ausgerüstet werden. Das Polyurethan quillt durch den Kontakt mit Wasser leicht an, so daß das Hydrophobierungsmittel selektiv auf der Oberfläche des Polyurethan abgeschieden und dort sehr gut verankert wird. Durch anschließendes Trocknen und Verfilmen wird die Wirkung des Hydrophobierungsmittels noch verbessert.

Wird das Hydrophobierungsmittel nur in die Deckschicht des Films eingemischt oder auf diese aufgesprüht, erhält ausschließlich die der Haut abgewandte Oberfläche des Filmpflasters die gewünschten Effekte. Die Haftung der Klebemasse auf der gegenüberliegenden Oberfläche wird nicht beeinflußt.

Somit stellt sich ein weiteres bevorzugtes Verfahren zur Herstellung eines erfindungsgemäßen Filmpflasters wie folgt dar.

Zumindest eine Polyurethan-Dispersion wird auf einem geprägten wasserfesten Siliconoder Polypropylen-beschichteten Papier oder Folie ausgestrichen. Der Verbund wird getrocknet.

Der sich daraus ergebene Polyurethanfilm wird einseitig mit einer wäßrige, ein Hydrophobierungsmittel insbesondere zu einem Anteil von bis zu 40 Gew.-% enthaltenden Lösung besprüht der Polyurethan-Film auf der der besprühten Seite gegenüberliegenden Seite mit einer Haftklebemasse beschichtet, die Haftklebemasse gegebenenfalls mit einer Wundabdeckung und einem klebstoffabweisenden Trägermaterial versehen wird und das wasserfeste Silicon- oder Polypropylen-beschichtete Papier oder die Folie entfernt.

Weiter bevorzugt eingesetzte Dispersionen enthalten Hydrophobierungsmittel zu einem Anteil von bis zu 30 Gew.-% gegebenenfalls bis zu 20 Gew.-%.

Hydrophobierungsmittel zur Verbesserung von Gleitfähigkeit, Wasser- und Schmutz-Abweisung können von ihrem chemischen Aufbau Fluorcarbon-Polymere, Silicone oder Kohlenwasserstoffe sein.

Geeignete Fluorcarbon-Polymere sind beispielsweise unter den Bezeichnungen Baygard (Firma Bayer AG, Leverkusen), Zonyl (Firma DuPont, Bad Homburg), Stralin (Weserland Textilchemie, Hannover), und Unidyne (Daikin Chemicals, Düsseldorf) bei den jeweiligen Anbietem erhältlich. Als Silicone lassen sich beispielsweise Dow Coming 365 (Firma Dow Coming, Sophia Antipolis, Frankreich) oder Finish WS 60 E (Firma Wacker, 84489 Burghausen) einsetzen. Als Hydrophobierungsmittel auf Kohlenwasserstoff-Basis sind beispielsweise Nalan GN, Nalan W (Firma DuPont, Bad Homburg) oder Perlit-Typen wie 40178, SE oder SI-SW (Bayer AG, Leverkusen) geeignet.

Durch Kombination von fluorierten und nicht-fluorierten Hydrophobierungsmitteln läßt sich die benötigte Menge an fluoriertem Einsatzmaterial verringern.

Eine bevorzugte Folie ist etwa 10 bis 500 μm, vorzugsweise 20 bis 100 μm stark, das Gewicht entsprechend zwischen etwa 15 bis 600 g/m$^2$, vorzugsweise 15 bis 100 g/m$^2$, transparent, weist eine Höchstzugkraft längs zwischen etwa 2 bis 100 N/cm, vorzugsweise 5 bis 40 N/cm, und eine Reißdehnung längs zwischen etwa 100 bis 1000%, vorzugsweise über 450%, und eine Wasserdampfdurchlässigkeit von über 500 g/m$^2$ in 24h bei 38 °C und 95% rel. Feuchte nach DAB auf. Besteht die erfindungsgemäße Folie teilweise aus geschäumten Schichten, so kann die Dicke von 50 μm bis 2 mm betragen.

Als Haftklebemassen können handelsüblichen Klebmassen medizinischer Qualität eingesetzt werden.
Die Haftklebemasse auf der Polyurethanfolie weist beispielsweise bevorzugt eine Klebkraft auf Stahl von etwa 2 bis 4 N/cm auf, wobei das Prüfmaterial, da die Folie sehr dehnbar ist, für die Messung rückseitig mit einem unelastischen Klebefilm verstärkt werden muß. Die Messung selbst erfolgte in Anlehnung an DAB 9.

Auf seiner gegebenenfalls selbstklebend ausgerüsteten, später der Haut zugewandten Seite ist das erfindungsgemäße Folienpflaster über seine ganze Breite bis zum Gebrauch üblicherweise mit einem klebstoffabweisenden Trägermaterial, wie silikonisiertem Papier, abgedeckt. Dieses schützt die Selbstklebeschicht aus einer gut hautverträglichen Klebemasse, beispielsweise auf Acrylatbasis, die vorzugsweise im Transferverfahren aufgebracht worden ist, und stabilisiert zusätzlich das ganze Produkt. Die Abdeckung kann in bekannter Weise einstückig oder vorzugsweise zweiteilig ausgebildet sein.

Das Folienpflaster kann als solches verwendet werden, es kann jedoch auch zusätzlich mittig in geeigneter Breite eine übliche, saugende Wundauflage oder ein anderes funktionales Material, welche positive Auswirkungen auf die Heilung von Wunden oder Blasen haben, aufgebracht sein, so daß es direkt als Wundverband eingesetzt werden kann. Ein derartiger Verband mit Rundum-Verklebung ist besonders vorteilhaft, da er keimdicht und wasserfest ist

Das Produkt kann zur Sterilisation nach Standard-Verfahren verpackt und γ-bestrahlt werden.

In einer alternativen Ausführungsform des Filmpflasters umfaßt dieses einen mindestens zweischichtigen elastischen Film, wobei die erste Schicht aus einem elastischen Polyurethan-Film besteht, wobei der Polyurethan-Film mit Hydrophobierungsmitteln auf Fluorcarbon-, Silicon- oder Kohlenwasserstoff-Basis ausgerüstet ist, wobei die erste Schicht strukturiert ist und wobei die Oberfläche der unteren Schicht gegebenenfalls mit einer Haftklebemasse beschichtet ist

in einer ersten bevorzugten Ausführungsform ist zwischen der oberen und der unteren Schicht zumindest eine weitere Schicht vorhanden, die u. a. zur Verbesserung der Keimdichtigkeit dient.

Erfindungsgemäß ist unter der Struktur der oberen Schicht eine erhabene Musterung zu verstehen, so daß die Schicht nicht flächenhaft ausgebildet ist, sondern dreidimensionale Erhebungen beziehungsweise Vertiefungen aufweist Die obere Schicht kann auch in einer bevorzugten Ausführungsform aus einzelnen diskreten (also voneinander getrennten) Segmenten bestehen.

Besonders gut läßt sich ein derartiges Filmpflaster herstellen, indem eine Polyurethan-Dispersion auf einem geprägten wasserfesten Silicon- oder Polypropylen-beschichteten Papier oder Folie ausgestrichen wird, so daß sich eine strukturierte Schicht ergibt, insbesondere bestehend aus einzelnen voneinander getrennten Segmenten,
der Verbund getrocknet wird,
eine zweite und gegebenenfalls dritte Polyurethan-Dispersion auf der ersten ausgestrichen werden,
der Verbund getrocknet wird,
der Polyurethan-Film auf der strukturierten Seite mit einer Hydrophobierungsmittel-Dispersion mit einem Feststoffgehalt von bis zu 40 Gew.-% bestrichen oder besprüht wird,
der behandelte Film getrocknet wird,
der sich daraus ergebene Polyurethanfilm mit einer Haftklebemasse beschichtet wird,
die Haftklebemasse gegebenenfalls mit einer Wundabdeckung und einem klebstoffabweisenden Trägermaterial versehen wird und

das wasserfeste Silicon- oder Polypropylen-beschichtete Papier oder die Folie entfernt wird.

Gegenstand und Inhalt der Erfindung soll anhand der nachfolgenden Beispiele erläutert werden, ohne damit die Erfindung in irgendeiner Weise beschränken zu wollen.

## Beispiel 1. Ausrüstung mit Baygard AFF

Durch Verdünnen von kommerziell erhältlichem Baygard AFF (Bayer AG, Leverkusen) wurde eine 1% Lösung hergestellt. Musterstücke eines hydrophilen Polyurethan-Films, hergestellt aus Impranil DLH und Impranil DLN im Verhältnis 1:1, wurde in die Lösung eingetaucht und dort einige Minuten unter leichtem Schütteln aufbewahrt. Danach wurde der Film aus dem Bad entfernt und in einem Trockenschrank bei 120° C getrocknet und anschließend für 1 min bei 160° C verfilmt.

## Beispiel 2. Ausrüstung mit 7-9931 (Dow Corning)

Kommerziell erhältliches Hydrophobierungsmittel auf Silicon-Basis (7-9931, Dow Corning) wurde durch Zusatz von demineralisiertem Wasser auf 7,5 % Feststoffgehalt verdünnt

Musterstücke eines hydrophilen Polyurethan-Films, hergestellt aus Impranil DLH und Impranil DLN im Verhältnis 1:1, wurde in die Lösung eingetaucht und dort dreißig Minuten unter leichtem Schütteln aufbewahrt.

Anschließend wurde der Film aus dem Bad entfernt und 10 min bei 120° C getrocknet

## Beispiel 3. Ausrüstung mit Nalan GN (Du Pont)

Kommerziell erhältliches Hydrophobierungsmittel auf Kohlenwasserstoff-Basis (Nalan GN) wurde durch mit demineralisiertem Wasser auf 6% Feststoffgehalt verdünnt.

Musterstücke eines hydrophilen Polyurethan-Films, hergestellt aus Impranil DLH und Impranil DLN im Verhältnis 1:1, wurde in die Lösung eingetaucht und dort dreissig Minuten unter leichtem Schütteln aufbewahrt

Anschließend wurde der Film aus dem Bad entfernt und 15 min bei 120° C getrocknet.

Nachfolgende Tabelle führt erfindungsgemäße, mit Hydr-ophobierungsmitteln behandelte hydrophile Polyurethanfolien auf. Die Zusammensetzung des Films wird zuerst aufgeführt. Die Spalte "Hydrophobierung" beschreibt Art und Menge der eingesetzten Hydrophobierungsmittel. Die Reibungskoeffizienten μ, bestimmt nach DIN 53 375, und die Oberflächenenergie, bestimmt durch Messung der Randwinkel von verschiedenen Flüssigkeiten, sind in den jeweiligen Spalten aufgeführt.

Tabelle 2:

| Eigenschaften von erfindungsgemäßen Polyurethanfilmen | | | |
| --- | --- | --- | --- |
| Folie | Menge, bzw. Konzentration | Oberflächenenergie [mN/m] | Reibungskoeffizient μ |
| Vergleichsbeispiel 1 | - | n.b. | 1,5 |
| 1 + Xeroderm WF[1] | 2% | n.b. | 1,2 |
| 1 + Xeroderm WF[1] | 5% | n.b. | 1,2 |
| | | | |
| Vergleichsbeispiel 2 | - | 30 | 2,3 |
| 2 + Baygard AFF[1] | 1% | 21 | 1,8 |
| 2 + Stralin TFK 3[2] | 5% | 15 | 1,7 |
| 2 + Unidyne TG 561[3] | 2,5% | 7 | 1,5 |
| | | | |
| Vergleichsbeispiel 3 | - | 30 | 1,6 |

(1) Bayer AG, Leverkusen

(2) Wesenand Textilchemie

(3) Daikin Chemicals

Tabelle 2:  (fortgesetzt)

| Eigenschaften von erfindungsgemäßen Polyurethanfilmen | | | |
|---|---|---|---|
| Folie | Menge, bzw. Konzentration | Oberflächenenergie [mN/m] | Reibungskoeffizient μ |
| 3 + Finish WS6OE[4] | 4% | 12 | 0,7 |
| 3 + Silicon 365[5] | 18% | 31 | 0,9 |
| 3 + Silicon 7.9931[5] | 8% | 15 | 0,6 |
| 3 + Nalan W[6] | 2,5% | 11 | 0,8 |
| 3 + Nalan GN[6] | 6% | 12 | 1,1 |

(4) Wacker Chemie

(5) Dow Coming

(6) DuPont

Obige Tabelle zeigt die Bedeutung des Parameters Oberflächenenergie bei der Herstellung eines Films mit definierten Oberflächeneigenschaften.

Dem Fachmann ist unmittelbar einsichtig, daß oben genannte Einflußgrößen auch geeignet sind, Polyurethanfilme mit gewünschter Anmutung und Griff herzustellen. Für Anwendungen, bei denen weniger die Gleiteigenschaften als die Anmutung im Vordergrund steht, können Eigenschaften wie Anschmiegsamkeit und angenehme Haptik durch gezielte Optimierung der genannten Parameter verbessert werden.

**Patentansprüche**

1.  Filmpflaster bestehend aus zumindest einem elastischen Polyurethan-Film, der auf der der Haut abgewandten Oberflächenseite mit Hydrophobierungsmitteln auf Fluorcarbon-, Silicon- oder Kohlenwasserstoff-Basis ausgerüstet ist und auf der Haut zugewandten Seite mit einer Haftklebemasse beschichtet ist.

2.  Filmpflaster nach Anspruch 1, **dadurch gekennzeichnet, dass** das Filmpflaster über seine ganze Breite bis zum Gebrauch mit einem klebstoffabweisenden Trägermaterial, wie silikonisiertes Papier, abgedeckt ist.

3.  Filmpflaster nach den Ansprüchen 1 oder 2, **dadurch gekennzeichnet, dass** das Filmpflaster mittig in geeigneter Breite eine übliche, saugende Wundauflage oder ein anderes funktionales Material, welche positive Auswirkungen auf die Heilung von Wunden oder Blasen haben, aufweist.

4.  Verfahren zur Herstellung eines Filmpflasters gemäß Anspruch 1, wobei zumindest eine Polyurethan-Dispersionen, enthaltend ein Hydrophobierungsmittel, insbesondere zu einem Anteil von bis zu 5 Gew.-%, auf einem geprägten wasserfesten Silicon- oder Polypropylen-beschichteten Papier oder Folie ausgestrichen wird, der Verbund getrocknet wird, der sich daraus ergebende gegebenenfalls mehrlagige Polyurethanfilm mit einer Haftklebemasse beschichtet wird, die Haftklebemasse gegebenenfalls mit einer Wundabdeckung und einem klebstoffabweisenden Trägermaterial versehen wird und das wasserfeste Silicon- oder Polypropylen-beschichtete Papier oder die Folie entfernt wird.

5.  Verfahren zur Herstellung eines Filmpflasters gemäß Anspruch 1, wobei zumindest eine Polyurethan-Dispersionen auf einem geprägten wasserfesten Siliconoder Polypropylen-beschichteten Papier oder Folie ausgestrichen wird, der Verbund getrocknet wird, der sich daraus ergebene Polyurethanfilm einseitig mit einer wässrigen, ein Hydrophobierungsmittel, insbesondere zu einem Anteil von bis zu 40 Gew.-%, enthaltenden Lösung besprüht wird, der Polyurethan-Film auf der der besprühten Seite gegenüberliegenden Seite mit einer Haftklebemasse beschichtet wird, die Haftklebemasse gegebenenfalls mit einer Wundabdeckung und einem klebstoffabweisenden Trägermaterial versehen wird und das wasserfeste Silicon- oder Polypropylen-beschichtete Papier oder die Folie entfernt wird.

6.  Filmpflaster umfassend einen zweischichtigen elastischen Film, wobei die erste Schicht aus einem elastischen Polyurethan-Film besteht, wobei der Polyurethan-Film mit Hydrophobierungsmitteln auf Fluorcarbon-, Silicon- oder Kohlenwasserstoff-Basis ausgerüstet ist, wobei die erste Schicht partiell aufgetragen ist und wobei die Oberfläche der unteren Schicht mit einer Haftklebemasse beschichtet ist.

7.  Filmpflaster nach Anspruch 6, **dadurch gekennzeichnet, dass** die obere Schicht aus einzelnen voneinander getrennten Segmenten besteht.

8.  Filmpflaster nach den Ansprüchen 6 oder 7, **dadurch gekennzeichnet, dass** zwischen der ersten und der zweiten Schicht zumindest eine weitere Schicht vorhanden ist.

9.  Verfahren zur Herstellung eines Filmpflasters gemäß Anspruch 6, wobei
    eine Polyurethan-Dispersionen, enthaltend ein Hydrophobierungsmittel, insbesondere zu einem Anteil von bis zu 5 Gew.-%, auf einem geprägten wasserfesten Silicon- oder Polypropylen-beschichteten Papier oder Folie ausgestrichen wird, so dass sich eine strukturierte Schicht ergibt, insbesondere bestehend aus einzelnen voneinander getrennten Segmenten, der Verbund getrocknet wird, eine zweite und gegebenenfalls dritte Polyurethan-Dispersion auf der ersten ausgestrichen werden, der Verbund getrocknet wird, der sich daraus ergebene Polyurethanfilm gegebenenfalls mit einer Haftklebemasse beschichtet wird, die Haftklebemasse gegebenenfalls mit einer Wundabdeckung und einem klebstoffabweisenden Trägermaterial versehen wird und das wasserfeste Silicon- oder Polypropylen-beschichtete Papier oder die Folie entfernt wird.

10. Verwendung eines Filmpflasters nach einem der Ansprüche 1, 2, 3, 6, 7 oder 8 zur Abdeckung von Wunden und Verhütung oder Behandlung von Blasen.

**Claims**

1.  A film plaster, comprising at least one elastic polyurethane film which is provided, on the surface side facing away from the skin, with fluorocarbon-, silicone- or hydrocarbon-based water repellents and is coated, on the side facing the skin, with a pressure-sensitive adhesive composition.

2.  The film plaster as claimed in claim 1, covered over its entire width, up until the time of use, with an antiadhesive carrier material, such as siliconized paper.

3.  The film plaster as claimed in claim 1 or 2, comprising centrally in an appropriate width a customary, absorbent wound contact material or another functional material having beneficial effects on the healing of wounds or blisters.

4.  A process for producing a film plaster as claimed in claim 1, which comprises applying at least one polyurethane dispersion comprising a water repellent in particular in a fraction of up to 5% by weight to an embossed, water-resistant, silicone- or polypropylene-coated paper or film, drying the composite, coating the resulting, optionally multi-ply, polyurethane film with a pressure-sensitive adhesive composition, providing the pressure-sensitive adhesive composition, if desired, with a wound pad and an adhesive repellent backing material, and removing the water-resistant, silicone- or polypropylene-coated paper or film.

5.  A process for producing a film plaster as claimed in claim 1, which comprises applying at least one polyurethane dispersion to an embossed, water-resistant, silicone- or polypropylene-coated paper or film, drying the composite, spraying the resulting polyurethane film on one side with an aqueous solution comprising a water repellent, in particular in a fraction of up to 40% by weight, coating the polyurethane film on the side opposite the sprayed side with a pressure-sensitive adhesive composition, providing the pressure-sensitive adhesive composition, if desired, with a wound pad and an adhesive repellent backing material, and removing the water-resistant, silicone- or polypropylene-coated paper or film.

6.  A film plaster comprising a two-layer elastic film, the first layer being composed of an elastic polyurethane film, said polyurethane film being treated with fluorocarbon-, silicone- or hydrocarbon-based water repellents, the first layer being applied partially and the surface of the lower layer being coated with a pressure-sensitive adhesive composition.

7.  The film plaster as claimed in claim 6, wherein the upper layer is composed of individual, separate segments.

8.  The film plaster as claimed in claim 6 or 7, wherein between the first layer and second layers there is at least one further layer.

**9.** A process for producing a film plaster as claimed in claim 6, which comprises applying a polyurethane dispersion comprising a water repellent in particular in a fraction of up to 5% by weight to an embossed, water-resistant, silicone- or polypropylene-coated paper or film so as to give a structured layer consisting in particular of individual, separate segments, drying the composite, applying a second and, if desired, a third polyurethane dispersion to the first, drying the composite, coating the resulting polyurethane film, if desired, with a pressure-sensitive adhesive composition, providing the pressure-sensitive adhesive composition, if desired, with a wound pad and an adhesive repellent backing material, and removing the water-resistant, silicone- or polypropylene-coated paper or film.

**10.** The use of a film plaster as claimed in any of claims 1, 2, 3, 6, 7 and 8 for covering wounds and preventing or treating blisters.

**Revendications**

**1.** Pansement pelliculaire constitué au moins un film de polyuréthanne élastique qui est revêtu sur le côté de sa surface qui n'est pas tourné vers la peau d'agents d'imperméabilisation à base d'hydrocarbures fluorés, de silicone ou d'hydrocarbure, et qui est revêtu d'une pâte adhésive sur son côté tourné vers la peau.

**2.** Pansement pelliculaire selon la revendication 1, **caractérisé en ce que** le pansement pelliculaire est recouvert sur toute sa largeur et jusqu'à son utilisation d'un matériau de support non adhésif, par exemple du papier siliconé.

**3.** Pansement pelliculaire selon la revendication 1 ou 2, **caractérisé en ce que** le pansement pelliculaire présente en son milieu et sur une largeur appropriée une couche thérapeutique habituelle et aspirante ou un autre matériau fonctionnel qui ont des effets positifs sur la cicatrisation de blessures ou d'ampoules.

**4.** Procédé pour la fabrication d'un pansement pelliculaire selon la revendication 1, dans lequel au moins une dispersion de polyuréthanne qui contient un agent d'imperméabilisation, en particulier à une teneur qui peut atteindre 5% en poids, est appliquée sur un papier ou une feuille gaufré(e), résistant(e) à l'eau et revêtu(e) de silicone ou de polypropylène, l'ensemble étant séché, le film de polyuréthanne le cas échéant en plusieurs couches ainsi obtenu étant revêtu d'une pâte adhésive, la pâte adhésive étant le cas échéant dotée d'un recouvrement de blessure et d'un matériau de support non adhésif, et le papier ou la feuille résistant(e) à l'eau et revêtu(e) de silicone ou de polypropylène étant enlevé(e).

**5.** Procédé pour la fabrication d'un pansement pelliculaire selon la revendication 1 dans lequel au moins une dispersion de polyuréthanne est appliquée sur un papier ou une feuille gaufré(e), résistant(e) à l'eau et revêtu(e) de silicone ou de polypropylène, l'ensemble étant séché, une solution aqueuse qui contient un agent d'imperméabilisation en particulier à une teneur qui peut atteindre 40% en poids étant pulvérisée sur le film de polyuréthanne ainsi obtenu, le film de polyuréthanne étant revêtu d'une pâte adhésive sur son côté opposé au côté revêtu par pulvérisation, la pâte adhésive étant le cas échéant dotée d'un recouvrement de blessure et d'un matériau de support non adhésif et le papier ou la feuille résistant(e) à l'eau et revêtu(e) de silicone ou de polypropylène étant enlevé(e).

**6.** Pansement pelliculaire qui comprend un film élastique en deux couches, la première couche étant constituée d'un film de polyuréthanne élastique, le film de polyuréthanne étant doté d'agents d'imperméabilisation à base d'hydrocarbures fluorés, de silicone ou d'hydrocarbures, la première couche étant appliquée partiellement et la surface de la couche inférieure étant dotée d'une pâte adhésive.

**7.** Pansement pelliculaire selon la revendication 6, **caractérisé en ce que** la couche supérieure est constituée de segments individuels séparés les uns des autres.

**8.** Pansement pelliculaire selon la revendication 6 ou 7, **caractérisé en ce qu'**au moins une autre couche est prévue entre la première et la deuxième couche.

**9.** Procédé de fabrication d'un pansement pelliculaire selon la revendication 6, dans lequel une dispersion de polyuréthanne qui contient un agent d'imperméabilisation en particulier à une teneur qui peut atteindre 5% en poids est appliquée sur un papier ou une feuille gaufré(e); résistant(e) à l'eau, revêtu(e) de silicone ou de polypropylène de manière à obtenir une couche structurée qui est en particulier constituée de segments individuels séparés les uns des autres, l'ensemble étant séché, une deuxième et le cas échéant une troisième dispersion de polyuréthanne étant appliquées sur la première, l'ensemble étant séché, le film de polyuréthanne ainsi obtenu étant le cas échéant revêtu d'une pâte adhésive, la pâte adhésive étant le cas échéant dotée d'un recouvrement de blessure et d'un matériau de support non adhésif, le papier ou la feuille résistant(e) à l'eau et revêtu(e) de silicone ou de polypropylène étant enlevé(e).

10. Utilisation d'un pansement pelliculaire selon l'une quelconque des revendications 1, 2, 3, 6, 7 ou 8 pour recouvrir des blessures et pour prévenir ou traiter des ampoules.